# EUROPEAN PATENT SPECIFICATION

(11) **EP 1 638 415 B1**
(45) Date of publication and mention of the grant of the patent: **15.12.2010**
(21) Application number: 04740059.3
(22) Date of filing: 18.06.2004
(51) Int. Cl.: A23L 1/29, A23L 1/30, A23L 1/305, A61K 35/74

(54) **INFANT OR FOLLOW-ON FORMULA**
SÄUGLINGSNÄHRPRÄPARAT ODER FOLGEMILCH
FORMULE INFANTILE

(30) Priority: 23.06.2003 EP 03014055
(43) Date of publication of application: 29.03.2006
(73) Proprietor: Nestec S.A., 1800 Vevey (CH)
(72) Inventor: SECRETIN, Marie-Christine, 1807 Blonay (CH)
(74) Representative: Dixon, Sarah
(86) International application number: PCT/EP2004/006614
(87) International publication number: WO 2004/112508

(56) References cited:
- EP-A- 0 880 902
- EP-A- 0 904 784
- EP-A- 1 048 226
- EP-A- 1 228 707
- WO-A-02/15719
- WO-A-02/060276
- DE-U- 20 202 562
- US-A- 5 916 621
- US-A1- 2002 004 527
- US-A1- 2003 017 192
- US-A1- 2003 077 255
- OGATA T ET AL: "EFFECT OF BIFIDOBACTERIUM LONGUM BB536 YOGHURT ADMINISTRATION ON THE INTESTINAL ENVIRONMENT OF HEALTHY ADULTS" MICROBIAL ECOLOGY IN HEALTH & DISEASE, CHICHESTER, GB, vol. 11, no. 1, March 1999 (1999-03), pages 41-46, XP009023764
- GRILL J P ET AL: "Bifidobacteria and probiotic effects: action of Bifidobacterium species on conjugated bile salts." CURRENT MICROBIOLOGY 1995 CORRESPONDENCE (REPRINT) ADDRESS, J. BALLONGUE, LAB. DE CHIMIE BIOL., INST. HENRY TISSIER, UNIV. DE NANCY I, BP 239, 54506 VANDOEUVRE LES NANCY, FRANCE, vol. 31, no. 1, 1995, page 23, XP008036111
- ISOLAURI E ET AL: "Probiotics in the management of atopic eczema" CLINICAL AND EXPERIMENTAL ALLERGY, vol. 30, no. 11, November 2000 (2000-11), pages 1604-1610, XP002298117 ISSN: 0954-7894
- SAXELIN M: "LACTOBACILLUS GG - A HUMAN PROBIOTIC STRAIN WITH THOROUGH CLINICAL DOCUMENTATION" FOOD REVIEWS INTERNATIONAL, NEW YORK, NY, US, vol. 13, no. 2, 1997, pages 293-313, XP000199732
- KALLIOMAKI M ET AL: "Probiotics and prevention of atopic disease: 4-year follow-up of a randomised placebo-controlled trial" LANCET THE, LANCET LIMITED. LONDON, GB, vol. 361, no. 9372, 31 May 2003 (2003-05-31), pages 1869-1871, XP004428374 ISSN: 0140-6736
- MATSUMOTO M ET AL: "ADHESIVE PROPERTY OF BIFIDOBACTERIUM LACTIS LKM512 AND PREDOMINANT BACTERIA OF INTESTINAL MICROFLORA TO HUMAN INTESTINAL MUCIN" CURRENT MICROBIOLOGY, NEW YORK, NY, US, vol. 44, no. 3, 2002, pages 212-215, XP008024172 ISSN: 0343-8651
- BRUCK W M ET AL: "A two-stage continuous culture system to study the effect of supplemental alpha-lactalbumin and glycomacropeptide on mixed cultures of human gut bacteria challenged with enteropathogenic Escherichia coli and Salmonella serotype typhimurium." JOURNAL OF APPLIED MICROBIOLOGY, vol. 95, no. 1, 2003, pages 44-53, XP002261744 ISSN: 1364-5072

## Description

### Field of the invention

The present invention relates to a nutritional composition intended for infants and/or young children, as well as to methods for improving gastrointestinal comfort, developing a healthy gut microflora, and promoting the physical development of infants and/or young children by partly or fully feeding said infants or children with the aforementioned nutritional composition.

### Background of the invention

The composition of human milk serves as a valuable reference for improving infant formula. However, human milk contains living cells, hormones, active enzymes, immunoglobulins and components with unique molecular structures that cannot be replicated in infant formula. Unlike human milk, infant formula must remain stable on the shelf for up to thirty-six (36) months. These fundamental differences between human milk and infant formula often mandate differences in the composition to achieve similar clinical outcome.

The study of human milk components has stimulated many investigations into what constituents may be added to an improved infant formula. Greater knowledge of the composition of human milk affords the opportunity to design infant formulas that are closer in composition to human milk. However, it becomes increasingly apparent that infant formula can never exactly duplicate human milk. Many constituents in human milk are bioactive and because of synergies among these components, there is little reason to believe that the same compound would have the same bioactivity in infant formula. The likelihood of this possibility is further diminished when the impact of heat treatment for sterilization and long-term storage of the formula is considered.

The composition of human milk differs appreciably from that of other species and much attention has been paid to the various components. Several investigators have reported on the nucleotide content of milk from humans. Numerous publications have also discussed various lipid, oil or fat blends for use in an artificial nutritional for human infants.

EP 1 048 226 describes an infant formula comprising a lipid source, a carbohydrate source and a protein source. The protein source comprises the free amino acids arginine, tyrosine and histidine and a hydrolysed sweet whey fraction from which caseino-glyco-macropeptide has been removed. US 5, 916,621 discloses a whey protein dominant baby food with a reduced threonine content which may be obtained by using glycomacropeptide-free or glycomacropeptid-reduced whey powder or whey concentrate in the production of the baby food. US2003/077255 relates to the use of a combination of lactic acid bacteria and monomeric alpha-lactalbumin to treat gastric ulcers. EP 904784 is directed to a nutritional composition containing a minimum of three different probiotic strains with the intention of providing protection against infection all the way along the gastro-intestinal tract thus obviating the need to identify the type of micro-organism responsible for the infection. US2003/017192 is directed to the production of shelf-stable liquid products containing probiotics.

There is a need for new formulae, providing to the infant or the young child a nutritional contribution with a unique combination of protective nutrients, especially ensuring growth and metabolic patterns similar to those of breastfed infants, thus resulting in similar health characteristics in later childhood and adulthood.

### Summary of the Invention

The present invention therefore pertains to formulae intended both for infants and young children. The formula of the invention comprises comprising a source of proteins, a source of lipids, a source of carbohydrates and a probiotic, wherein at least 40% of the proteins are modified sweet whey proteins comprising no CGMP or reduced CGMP and the protein content of the formula is no more than 2g/100kcal.

The present invention further provides to a method of developing a healthy gut microflora in an infant or a young child consisting in fully or partly feeding the infant or child with the said formula.

### Detailed Description of the Invention

In the present specification, the hollowing words are given a definition that must be taken into account when reading and interpreting the description, examples and claims.

Infant: according to the Commission Directive 91/321/EEC of 14 May 1991 on infant formulae and follow-on formulae, article 1.2(a), the term "infants" means children under the age of 12 months. This definition is adopted in the present specification.

Young Children: according to the Commission Directive 91/321/EEC of 14 May 1991 on infant formulae and follow-on formulae, article 1.2(b), the term "young children"

means children aged between one and three years. This definition is adopted in the present specification.

Infant formulae: according to the Commission Directive 91/321/EEC of 14 May 1991 on infant formulae and follow-on formulae, article 1.2(c), the term "infant formula" means foodstuffs intended for particular nutritional use by infants during the first four to six months of life and satisfying by themselves the nutritional requirements of this category of persons. This definition is adopted in the present specification. It has to be understood that infants can be fed solely with infant formulas, or that the infant formula can be used by the carer as a complement of human milk. It is synonymous to the widely used expression "starter formula".

Follow-on formulae: according to the Commission Directive 91/321/EEC of 14 May 1991 on infant formulae and follow-on formulae, article 1.2(d), the term "follow-on formulae" means foodstuffs intended for particular nutritional use by infants aged over four months and constituting the principal liquid element in a progressively diversified diet of this category of persons. This definition is adopted in the present specification.

Probiotic: according to the paper Probiotics in Man and Animals, J. Appl Bacteriol. 66: 365-378, a probiotic is defined as a live microbial feed supplement which beneficially affects the host animal by improving its intestinal microbial balance.

According to a first aspect of the invention, there is provided a nutritional formula for infants (including a starter composition) or young children. As already mentioned, it is an object of the invention to provide a unique combination of protective nutrients ensuring growth and metabolic patterns similar to those of breastfed infants, with the intention of enabling similar health characteristics to be enjoyed in later childhood and adulthood.

Formulae of the invention present a reduced load on immature organs; and favour the natural growth of Bifidobacteria and other beneficial microflora in the large intestine as in breastfed infants.

Dietary protein provides the essential amino acids necessary for protein synthesis and growth and protein quality is as important as protein quantity. Until recently, it was thought that in order to supply enough of the essential amino acids, formulae based on cows' milk needed a protein content significantly higher than that of the reference human milk. The protein content of regular whey-adapted formulae ranges from 2.1 to 2.6 g per 100 kcal, whereas the content of human milk ranges from 1.4 to 1.8 g per 100 kcal. Excess protein intake may induce metabolic stress on infant organs that have not fully developed. Following paediatric recommendations for lowering protein density of infant formulae, clinical trials in infants fed formulae containing protein densities between 1.6 and 2.0 g / 100 kcal have been reported. However, these attempts to lower protein content in a formula using traditional cow's milk protein sources or mixing the currently available fractions - casein and whey -, although demonstrating the principle was conceivable, failed to reproduce all the indices of human milk protein metabolism or to ensure the satisfactory growth of infants. For instance, results have shown a global plasma amino acid pattern different to that of breast-fed infants, depressed plasma tryptophan levels, elevated plasma threonine levels, delay in growth, and higher energy intake suggesting an increased fat deposition which may be responsible for obesity in later life.

However, if the amino acid pattern of a cow's milk-based infant formula can be made closer to that of human milk, the protein content of such a formula can be lowered to resemble that of the reference. A protein mixture of unique amino acid composition allowing the adaptation of the quantity of protein to a level closer to the average content of human milk has been developed according to an aspect of the present invention.

The protein fraction in cows' milk is a mixture of several proteins, which all have a different amino acid profile. Caseino-glyco-macropeptide (CGMP) is a protein that is found in this fraction. It comes from the kappa-casein that is split up by proteolytic cleavage into 2/3 para-kappa-casein, an insoluble fraction that remains in the casein fraction and 1/3 CGMP, a soluble fraction that is found in the whey fraction. An original fractionation process of whey proteins has been developed and is explained in EP 880902; this process allows the removal of practically all the CGMP (a fraction rich in threonine and poor in tryptophan) from bovine whey thereby increasing the alpha-lactalbumin proportion (a fraction very rich in tryptophan). By combining this modified sweet whey (MSW) fraction with skim milk, and with the addition of some free L-histidine and L-arginine (in order to reach the minimum amounts of these amino acids required by EC Directive), the protein source of the formula according to the invention has an amino acid profile much closer to that of human milk, characterised in particular by comparable tryptophan and threonine levels, allowing the adaptation of its protein content to that of human milk.

The nutritional value of this protein mixture has been measured in rats. The results show (see table 1) that this formulation has a Protein Efficiency Ratio (PER), a nitrogen digestibility, a Biological Value (BV), and a Net Protein Utilisation (NPU) comparable to standard whey-adapted formulae.

**Table 1**

| **Nutritional parameters** | **Casein** | **standard whey-adapted formula** | **New formulation** |
|---|---|---|---|
| PER | 1.36 | 2.49 | 2.70 |
| Relative PER (casein = 100%) | 100.0 | 182.8 | 198.3 |
| Digestibility (%) | 96.7 | 92.8 | 91.4 |
| BV | 0.88 | 0.96 | 0.96 |
| NPU (%) | 85.4 | 88.8 | 87.5 |

Moreover, rats fed on a formulation containing MSW showed significant lower plasma threonine levels and increased plasma tryptophan levels, compared to rats fed on standard whey-adapted formulae.

The protein content of formulae according to the present invention is no more than 2g/100 kcal, more preferably less than 1.85, most preferably between 1.8 and 1.85 g/100 kcal. This level is in line with recent data assessing protein requirements during early life, which has shown that recommendations for optimal protein intakes are lower than they have been reported in the past.

To ensure optimal protein synthesis, and therefore optimal growth, essential and semi-essential (i.e. essential only during infancy) amino acids need to be supplied in the same quantities as in human milk. Formulae according to the invention are preferably either whey enriched (casein / whey ratio set around 40/60) or, more preferably, whey predominant (casein / whey ratio preferably set at 30/70 or even more, such as 20/80). A preferred amino acid profile for formulae according to the invention is comparable to that of human milk (see table 2).

**Table 2**

| **Amino acid (g / 16 g N)** | **Human milk** | | | **Invention (representative values)** |
|---|---|---|---|---|
| | **mean** | **lowest value** | **highest value** | |
| Isoleucine * | 6.4 | 5.7 | 6.8 | 5.8 |
| Leucine * | 11.5 | 11.0 | 11.9 | 11.9 |
| Lysine * | 7.9 | 7.4 | 8.4 | 10.0 |
| Methionine * | 1.7 | 1.3 | 2.1 | 2.5 |
| Cystine ** | 2.3 | 1.7 | 2.9 | 2.4 |
| Phenylalanine* | 4.6 | 4.2 | 5.1 | 4.6 |
| Tyrosine ** | 4.7 | 3.3 | 6.3 | 4.0 |
| Threonine * | 5.6 | 5.3 | 6.6 | 5.4 |
| Tryptophan * | 2.3 | 1.8 | 2.6 | 2.1 |
| Valine * | 6.8 | 5.9 | 8.0 | 5.9 |
| Arginine ** | 4.2 | 3.5 | 4.9 | 4.5 |
| Histidine ** | 2.8 | 2.4 | 3.8 | 2.5 |
| Alanine | 4.8 | 4.5 | 5.3 | 5.1 . |
| Aspartic acid | 10.4 | 10.1 | 10.8 | 11.1 |
| Glutamic acid | 19.6 | 17.6 | 22.7 | 19.7 |
| Glycine | 3.2 | 2.8 | 3.6 | 2.7 |
| Proline | 10.2 | 8.9 | 11.2 | 7.8 |
| Serine | 5.6 | 5.0 | 5.9 | 5.3 |

| | | | | |
|---|---|---|---|---|
| All values corrected to 40% AH₃ * essential amino acids ** semi-essential amino acids | | | | |

The proteins may be either intact or partially hydrolysed by a process such as that described in European Patent No 322589.

Preferably, the sole source of carbohydrates of the composition according to the present invention is lactose. Carbohydrates constitute an important source of energy in the diet of the newborn infant. Lactose is the natural carbohydrate in human milk. Most infants in good health can digest lactose adequately. Further, lactose is associated with stool acidity and the development of a Bifidobacteria and lactobacilli preponderant microflora in the large intestine similar to that of breastfed babies. This is thought to be important in suppressing the growth of undesirable bacteria in the large intestine. Moreover, lactose has been shown to enhance absorption and retention of calcium and probably other minerals. In a recent study, it has been shown that calcium absorption is 10% greater from a lactose-containing formula compared with the same formula in which the lactose was replaced by glucose polymers.

As previously mentioned, formulae according to the invention comprise at least one probiotic, in order to offer all infants, whatever their mode of delivery or their hygienic environment, the advantages of a protective intestinal flora.

Preferred probiotics are those which as a whole are safe, are L (+) lactic acid producing cultures and have acceptable shelf-life for products such as infant and follow-on formulae which are required to remain stable and effective for up to 36 months.

Examples of preferred probiotics are:-
*Bifidobacteriuna lactis,* first sold by Christian Hansen company;
*Streptococcus thermophilus* provided under the name TH4 by Chr. Hansen, Denmark;
*Lactobacillus paracasei rhamnosus* GG (ATCC 53103) provided by Valio Oy, Finland;
*Bifidobacterium longum BB536* provided by Morinaga Milk Industry Co. Ltd, Japan.

The probiotics according to the present aspect of the invention are preferably present in an amount of 10⁶ to 10⁹cfu/ grams of dry product, preferably 10⁶ to 10⁸ cfu/g, and even more preferably 2*10⁷ cfu/grams of dry product.

The composition according to the present invention comprises at least one probiotic strain but combinations of different strains may also be used, particularly in follow-on formulae. If such a combination is to be used, it will preferably include at least one Bifidobacteria and at least one Lactobacillus. A particularly preferred combination is Bifidobacterium longum BB536 and Lactobacillus paracasei rhamnosus GG.

The formulae of the present invention also comprise a source of lipids. Fat provides about half of the dietary energy and constitutes the major energy stores in the bodies of infants and young children. Presently, there is growing interest in the quality of the dietary lipid supply during infancy as a major determinant of growth, visual and neural development, and long-term health. Thus, the selection of the dietary lipid supply during early life is considered to be of great importance.

Because of the small size of their stomach and their limited tolerance of hypertonic foods, infants require a concentrated source of energy. Of the 3 nutrients supplying energy, fat provides 9 kcal per gram, i.e. more than twice the energy present in carbohydrates or proteins. Most experts recommend that in infant and follow-on formulae fat should supply from 30% to 55% of the total energy. Preferably, the fats used in the formulae of the invention are predominantly vegetable fats. However, whey and skim milk naturally contain traces of milk fat, and so a very small percentage of milk fat is likely to be present.

Fatty acid composition of the diet determines fatty acid composition of all tissues, including storage tissues. The fat blend used in the formulae of the invention therefore preferably has an overall fatty acid composition as close as possible to that of human milk, in order to ensure similar membrane plasticity and same mobilization of energy in case of increased needs. Thus, the preferred fat blend supplies the essential fatty acids (linoleic and α-linolenic acids), as well as adequate quantities of oleic acid, palmitic acid, lauric acid and myristic acid.

Human milk contains docosahexaenoic acid (DHA) and arachidonic acid (ARA) and thus breast-feeding provides infants with preformed LC-PUFAs. The DHA content of human milk varies considerably within populations and is strongly influenced by maternal diet. Globally, the DHA content of milk from mothers consuming Western diets ranges from 0.1 to 0.4%, with a mean of 0.25%, whereas in mothers consuming non-Western diets, the DHA content of milk is greater, ranging from 0.1 to 1.4%, with a mean of 0.6%. However, amounts of 0.2 to 0.3% are generally accepted as representative. The ARA content of human milk is less influenced by the diet than DHA. Globally, the ARA content of human milk from mothers consuming Western diets ranges from 0.2 to 0.7%, with a mean of 0.45%, whereas in mothers consuming non-Western diets, the ARA content ranges from 0.4 to 1.2%, with a mean of 0.6%. Both DHA and ARA levels are influenced by the duration of lactation and tend to decrease from colostrum to transitional and mature milk.

Accordingly, the lipid source of the present invention preferably also comprises at least one preformed LC-PUFA such as DHA. The source of the DHA may be a natural fish oil that also supplies eicosapentaenoic acid (EPA) with a DHA/EPA ratio > 4. Together with DHA, the lipid source may also include ARA, for example from fungal origin such as Mortierella alpina.

Formulae of the invention preferable have a reduced level of electrolytes compared to standard infant and follow-on formulas. For example, the Na/K ratio (mmol) may be around 0.4, the (Na+K)/Cl ratio (mmol) may be around 1.8, Na+K+Cl may be around 34 and (Na+K)-Cl may be around 10.

Formulae according to the present invention preferably have a low phosphate content. Preferably, the calcium content varies between 35 and 45 mg/100 mL, the phosphorus content varies between 15 and 25 mg/mL, and the Ca/P ratio is between 1.4 and 3.

The most preferred amounts are indicated in table 3 below.

**Table 3**

| | invention (mg/100mL) | cow's milk (mg/100mL) | breast milk-average values (mg/100mL) |
|---|---|---|---|
| Calcium | 41 | 120 | 30 |
| Phosphorus | 21 | 90 | 15 |
| Ca / P ratio | 2 | 1.3 | |

The formulae according to the invention may also supply semi-essential nutrients which may be needed in particular conditions (e. g. taurine, nucleotides, carnitine, selenium).

Taurine is a free amino acid, which is not used to build up protein molecules. It has been shown to be involved in many physiological functions, e.g., as a trophic factor in the development of the central nervous system, maintaining the structural integrity of the membrane, regulating calcium homeostasis, as an osmolyte, a neuromodulator, and a neurotransmitter. It also conjugates with bile acids to form bile salts (essential for micelle formation and fat absorption).

Nucleotides are non protein nitrogen compounds which contain three characteristic components: a nitrogenous base, a sugar (ribose or deoxy-ribose), and one or more phosphate groups. Total nucleotide content in human milk represents 2 to 5% of the non-protein nitrogen. Cow's milk contains lower concentrations of nucleotides than human milk and its nucleotide profile differs markedly from that of human milk. Addition of nucleotides in the present infant formula follows the physiological pattern of nucleotides levels in human milk, with a predominance of easily metabolised pyrimidines over less desirable purines: addition of nucleotides to the infant formula is safe. The levels of addition are within the range allowed by the European Union Scientific Committee for Food and the European Directive.

Carnitine is a particular nitrogenous compound, which belongs to a group of food factors known as vitamin-like nutrients. It performs a crucial role in the energy supply of tissues during foetal life and in the neonatal period by facilitating the transport of long chain fatty acids into the mitochondria where beta-oxidation occurs. Fatty acids are indeed not able to pass in free form through the mitochondrial wall; the transfer into the mitochondria is governed by at least three enzymatic systems, namely carnitine - palmitoyl transferases I and II and carnitine -translocase, in which carnitine participates. Thus, carnitine is required for proper lipid oxidation and carnitine deficiency or low carnitine intake can lead to impaired fat utilisation and altered lipid metabolism. Carnitine has also a role in other metabolic processes, such as ketogenesis, lypolysis, and the maintenance of thermogenesis and nitrogen metabolism. Moreover, carnitine has been shown to improve utilisation of medium chain triglycerides in infants. Newborns have relatively low carnitine reserves and a very low activity of the enzyme catalysing the last step in the carnitine synthesis. Thus newborns are particularly at risk of becoming carnitine-deficient in the absence of an adequate supply of exogenous carnitine. Carnitine is preferably added to infant formulae, in order to reach a level close to that of human milk.

Formulae according to the invention may be in powder form or a ready to drink liquid. In the case of a powder formula, the following feeding table (table 4) may be used as a guide. However, the quantities may be changed according to medical advice. The introduction of an infant formula should be carried out under medical supervision. The standard reconstitution of formulae according to the invention is 12.9%, i.e. 12.9 g powder for 90 mL of water, which gives a caloric density of 67 kcal/100mL.

**Table 4**

| | quantity per feed | | No. of feeds per day | |
|---|---|---|---|---|
| Age of infant | Previously boiled water (mL) | number. of measuring scoops | Formula | Others |
| 1^{st} and 2^{nd} weeks | 90 | 3 | 6 | - |
| 3^{rd} and 4^{th} weeks | 120 | 4 | 5 | - |
| 2^{nd} month | 150 | 5 | 5 | - |
| 3^{rd} and 4^{th} months | 180 | 6 | 5 | - |
| 5^{th} and 6^{th} months | 210 | 7 | 5 | - |
| from the 7^{th} month onwards | 210 | 7 | 4-3 | 1-2 |

In the case of a ready-to-drink liquid, special care needs to be taken to ensure that the probiotic does not accidentally come into contract with the liquid. Preferably, the probiotic is stored in powder form separate from the liquid, and is incorporated and homogenised into the liquid just before consumption, e.g. up to two hours before consumption.

Formulae according to the invention have been shown to provide nutritional benefits including a better protein utilisation, a plasma amino acid pattern close to that of breast-fed infants, and adequate growth rates. The improved amino acid profile of formulae according to the invention results in better protein utilisation, as shown by the higher percentage of nitrogen retention found in infants fed with a formula according to the invention as compared with infants fed a regular whey-adapted formula (see table 5). As a result, the total amount of total nitrogen remains uncharged.

**Table 5: nitrogen balance**

| | whey-adapted standard formulae | formulae according to the invention |
|---|---|---|
| absorption | 89.5% | 89.3% |
| retention | 32.2% | 39.6% |

Plasma amino acids in infants fed with a formula according to the invention have been shown to be closer to those of breast-fed infants compared to those in infants fed standard whey-adapted formulae. Furthermore, the protein content of formulae of the invention meets the needs of normal term infants during the first months of life without excessive energy intakes or increased body mass index. Still further, weight and length gains of infants fed with formulae according to the invention are comparable to breast-fed infants.

Formulae according to the invention present a reduced load on immature organs. Amino acids consumed in excess and not used for protein synthesis accumulate in the blood (hyperaminoacidemias) and are metabolised in the liver into urea which must be excreted through the kidneys, thus increasing kidney load. This unnecessary metabolic stress is well illustrated in infants fed standard whey-adapted formulae by plasma amino acid levels and plasma urea levels above those observed in breast-fed inputs. The lower protein content of formulae according to the invention obviates metabolic stress on infant immature organs due to excess dietary protein intake. This beneficial effect has been demonstrated: infants fed with a formula according to the invention have plasma urea nitrogen concentrations similar to those found in breast fed infants, and significantly lower than those found in the infants fed standard whey-adapted formulae. Plasma urea nitrogen is a very sensitive indicator of the adequacy of protein intake as higher levels than in breast-fed infants denote excess amino acids not utilised, whereas lower levels denote insufficient protein supply. In addiction, the modified sweet whey and probiotic(s) in formulae according to the invention may have a synergistic effect in promoting physical development particularly when LC-PUFA are also present.

According to another aspect of the present invention, there is provided a method of developing a healthy gut microflora in an infant or a young child consisting in fully or partly feeding said infant or child with a formula according to the invention.

The gut microflora composition and the population size of strains is reported to be mainly regulated by competition for nutrients and oxygen availability. It is thought that factors in breast milk may be bifidogenic thus explaining the ease with which a favourable microflora is established in breast fed infants. Bifidobacteria, when growing, use lactose as substrate to produce lactic and acetic acids that decrease the intestinal pH to 4-5. The low buffering capacity of breast milk would allow maintenance of such a low pH, which inhibits the development of anaerobic putrefactive bacteria and enables the proliferation of Bifidobacteria and lactobacilli that are acid-tolerant. Without wishing to be bound by theory, the inventor believes that there is a synergy between the protein source in the formulae of the present invention and the probiotic such that a microflora similar to that found in breast fed babies is rapidly established and maintained in infants fed a formula according to the invention.

The reduced phosphate content of formula according to the first object of the present invention optimises bone formation and, together with lactose and low protein content, creates optimal condition for an intestinal flora with a predominance of Bifidobacteria.

### Examples

The following examples are illustrative of some of the products and methods of making the same falling within the scope of the present invention. They are not to be considered in any way limitative of the invention. Changes and modifications can be made with respect to the invention. That is, the skilled person will recognise many variations in these examples to cover a wide range of formulas, ingredients, processing, and mixtures to rationally adjust the naturally occurring levels of the compounds of the invention for a variety of applications.

### Example 1

The following example of a preferred formula according to the invention is illustrative only.

| Nutrient | | per 100kcal | per litre |
|---|---|---|---|
| Energy (kcal) | | 100 | 670 |
| Protein (g) (Casein/Whey: 30/70) | | 1.83 | 12.3 |
| Total Fat (g) | | 5.3 | 35.7 |
| Of which | | | |
| | Linoleic acid (g) | 0.77 | 5.2 |
| | α-Linolenic acid (mg) | 95 | 640 |
| | DHA(mg) | 12 | 77 |
| | ARA | 12 | 77 |
| Lactose (g) | | 11.2 | 74.7 |
| Minerals (g) | | 0.37 | 2.5 |
| Na (mg) | | 23 | 150 |
| K (mg) | | 89 | 590 |
| Cl (mg) | | 64 | 430 |
| Ca (mg) | | 62 | 410 |
| P (mg) | | 31 | 210 |
| Mg (mg) | | 7 | 50 |
| Mn (µg) | | 8 | 50 |
| Se (µg) | | 2 | 13 |
| Vitamin A (µg RE) | | 105 | 700 |
| Vitamin D (µg) | | 1.5 | 10 |
| Vitamin E (mg TE) | | 0.8 | 5.4 |
| Vitamin K1 (µg) | | 8 | 54 |
| Vitamin C (mg) | | 10 | 67 |
| Vitamin B1 (mg) | | 0.07 | 0.47 |
| Vitamin B2 (mg) | | 0.15 | 1.0 |
| Niacin (mg) | | 1 | 6.7 |
| Vitamin B6 (mg) | | 0.075 | 0.50 |
| Folic acid (µg) | | 9 | 60 |
| Pantothenic acid (mg) | | 0.45 | 3 |
| Vitamin B12 (µg) | | 0.3 | 2 |
| Biotin (µg) | | 2.2 | 15 |
| Choline (mg) | | 10 | 67 |
| Fe (mg) | | 1.2 | 8 |
| I (µg) | | 15 | 100 |
| Cu (mg) | | 0.06 | 0.4 |
| Zn (mg) | | 0.75 | 5 |

| | | | |
|---|---|---|---|
| Bifidobacterium longum BB 536: 1 x 10⁷ cfu/gram of dry product Lactobacillus paracasei rhamnosus GG: 2 x 10⁷ cfu/gram of dry product | | | |

### Example 2

Over 100 infants whose mothers have elected not to breast feed after the 14^{th} day of their life are enrolled in a randomised, controlled, double blind, multi-centre clinical trial of two groups. The first group is fed the formula of Example 1 and the second group is fed a similar formula but without probiotics. The following measurements, which are recognised parameters to assess the physical growth of infants, are made over 112 days (16 weeks):-mean weight gain in g/day, recumbent length, head circumference. Measurements are taken on recruitment then at 5 weeks, 8 weeks, 12 weeks and 16 weeks (in each case +/- 1 week) as follows:
Weight (to nearest 10 grams): infants are weighed without clothing on electronic weighing scales. The same scales are used for all infants at all visits. The electronic weighing scales are calibrated as per the manufacturer's recommendations at the start of the study and every 3 months thereafter until the end of the study.

Recumbent length (to nearest 1 mm): infants are measured using a standardised length board. At least two people are present to maintain proper body alignment and full body extension with feet flexed.

Head circumference (to nearest 1 mm): obtained using a standard non-elastic, plastic coated measuring tape. The measurement is taken approximately 2.5 cm above the eyebrows, directly over the largest circumference of the skull.

In addition, regular observations are made of digestive tolerance by observing stool characteristics, incidence of vomiting and regurgitation, frequency and duration of colic as follows:-
3 days after recruitment
3 days before and after anthropometric measurements at 5 weeks, 8 weeks and 12 weeks
3 days before final anthropometric measurements at 16 weeks

In addition, frequency of episodes of morbidity are noted (number of times seen by healthcare professionals plus episodes of ill health, particularly gastrointestinal conditions) are noted. These observations are made by the infants' care givers in a diary provided for the purpose.

It is found that infants fed the formula of the invention display satisfactory physical development coupled with improved gastrointestinal comfort (as demonstrated by the observations of digestive tolerance detailed above) when compared with the control group.

## Claims

1. Infant or follow-on formula comprising a source of proteins, a source of lipids, a source of carbohydrates and a probiotic, wherein at least 40% of the proteins are modified sweet whey proteins comprising no CGMP or reduced CGMP, and the protein content of the formula is no more than 2g/100Kcal.

2. Formula according to claim 1 wherein at least 60% of the proteins are modified sweet whey proteins comprising no CGMP or reduced CGMP.

3. Formula according to claim 1 or 2, wherein the probiotic is a Bifidobacteria or a Lactobacillus.

4. Formula according to claim 3 wherein the Bifidobacteria is Bifidobacterium longum BB 536.

5. Formula according to claim 3 wherein the Lactobacillus is Lactobacillus paracasei rhamnosus GG.

6. Formula according to claim 1 or 2 which contains both a Bifidobacterium and a Lactobacillus.

7. Formula according to claim 6 wherein the Bifidobacteria is Bifidobaeterium longum BB 536 and the Lactobacillus is Lactobacillus paracasei rhamnosus GG.

8. Formula according to any preceding claim wherein the proteins are intact.

9. Formula according to any of claims 1 to 7 wherein the proteins are partially hydrolysed.

10. Formula according to any preceding claim wherein the proteins are present in a maximiun proportion 1.85, most preferably between 1.8 and 1.85 g/100 kcal.

11. Formula according to preceding claim wherein the whey proteins with reduced CGMP represent at least 60% of the total proteins, preferably at least 70% of the total proteins.

12. Formula according to any preceding claim further comprising at least one LC-PUFA.

13. Formula according to claim 12 wherein the LC-PUFA comprises DHA, associated or not with ARA.

14. Formula according to one of claims 1 to 13 further having a low amount of electrolytes.

15. Use of a source of proteins, a source of lipids, a source of carbohydrates and at least one probiotic, wherein at least 40% of the proteins are whey proteins comprising no CGMP or reduced CGMP and the protein content of the formula is no more than 2 g/100 kcal in the manufacture of a formula for developing a healthy gut microflora in an infant or a young child

## Patentansprüche

1. Säuglings- oder Folge-Nährpräparat, das eine Proteinquelle, eine Lipidquelle, eine Kohlehydratquelle und ein Probiotikum aufweist, wobei zumindest 40 % der Proteine modifizierte Süßmolkeproteine sind, die kein CGMP oder reduziertes CGMP enthalten und wobei der Proteingehalt des Nährpräparats nicht mehr als 2 g/100 kcal ausmacht.

2. Nährpräparat nach Anspruch 1, wobei zumindest 60 % der Proteine modifizierte Süßmolkeproteine sind, die kein CGMP oder reduziertes CGMP aufweisen.

3. Folge-Nährpräparat nach Anspruch 1 oder 2, wobei das Probiotikum ein Bifidobakterium oder ein Lactobacillus ist.

4. Folge-Nährpräparat gemäß Anspruch 3, wobei das Bifidobakterium *Bifidobacterium longum* BB 536 ist.

5. Folge-Nährpräparat gemäß Anspruch 3, wobei der Lactobacillus *Lactobacillus paracasei rhamnosus* GG ist.

6. Folge-Nährpräparat gemäß Anspruch 1 oder 2, das sowohl ein Bifidobakterium als auch einen Lactobacillus enthält.

7. Nährpräparat gemäß Anspruch 6, wobei das Bifidobakterium *Bifidobacterium longum BB 536* und der Lactobacillus das *Lactobacillus Paracasei Rhamnosus* GG ist.

8. Nährpräparat nach irgendeinem der vorstehenden Ansprüche, wobei die Proteine intakt sind.

9. Nährpräparat nach irgendeinem der Ansprüche 1 bis 7, wobei die Proteine teilweise hydrolysiert sind.

10. Nährpräparat nach irgendeinem der vorstehenden Ansprüche, wobei die Proteine mit einem Maximalanteil von 1,85, am meisten bevorzugt zwischen 1,8 und 1,85 g/100 kcal vorliegen.

11. Nährpräparat gemäß dem vorstehenden Anspruch, wobei die Molkeproteine mit reduziertem CGMP zumindest 60 % der Gesamtproteine, bevorzugt zumindest 70 % der Gesamtproteine ausmachen.

12. Nährpräparat gemäß irgendeinem der vorstehenden Ansprüche, das ferner zumindest eine LC-PUFA aufweist.

13. Nährpräparat gemäß Anspruch 12, wobei die LC-PUFA DHA aufweist, die mit ARA verbunden oder nicht verbunden ist.

14. Nährpräparat nach einem der Ansprüche 1 bis 13, das ferner einen niedrigen Gehalt an Elektrolyten aufweist.

15. Verwendung einer Proteinquelle, einer Lipidquelle, einer Kohlehydratquelle und zumindest eines Probiotikums, wobei zumindest 40 % der Proteine Molkeproteine sind, die kein CGMP oder reduziertes CGMP aufweisen und wobei der Proteingehalt des Nährpräparats nicht mehr als 2 g/100 kcal beträgt, zur Herstellung eines Nährpräparats zur Entwicklung einer gesunden Darmflora eines Säuglings oder eines Kleinkindes.

## Revendications

1. Formule infantile comprenant une source de protéines, une source de lipides, une source de glucides et un probiotique, où au moins 40% de protéines sont des protéines modifiées de lactosérum doux ne comprenant pas de cGMP ou un taux réduit de cGMP et le contenu en protéines de ladite formule n'est pas plus de 2g/ 100 kcal.

2. Formule selon la revendication 1, où au moins 60% de protéines sont des protéines modifiées de lactosérum doux ne comprenant pas de cGMP ou un taux réduit de cGMP.

3. Formule selon la revendication 1 ou 2, où le probiotique est un Bifidobacterium ou un Lactobacille.

4. Formule selon la revendication 3, où le Bifidobacterium est Bifidobacterium longum BB536.

5. Formule selon la revendication 3, où le Lactobacille est Lactobacillus paracasei rhamnosus GG.

6. Formule selon la revendication 1 ou 2, qui comprend un Bifidobacterium et un Lactobacille.

7. Formule selon la revendication 6, où le Bifidobacterium est Bifidobacterium longum BB536 et le Lactobacille est Lactobacillus paracasei rhamnosus GG.

8. Formule selon l'une quelconque des revendications précédentes où les protéines sont intactes.

9. Formule selon l'une quelconque des revendications 1 à 7 où les protéines sont partiellement hydrolysées.

10. Formule selon l'une quelconque des revendications précédentes où les protéines sont présentes à un taux maximal de 1,85, plus préférablement entre 1,8 et 1,85 g/100 kcal.

11. Formule selon la revendication précédente où les protéines de lactosérum ayant un taux réduit de cGMP représentent au moins 60% des protéines totales, préférablement au moins 70% des protéines totales.

12. Formule selon l'une quelconque des revendications précédentes comprenant en outre au moins un LC-PUFA.

13. Formule selon la revendication 12, où le LC-PUFA comprend DHA, associé ou non avec ARA.

14. Formule selon l'une des revendications 1 à 13 ayant en outre un taux bas en électrolytes.

15. Utilisation d'une source de protéines, une source de lipides, une source de glucides et au moins un probiotique, où au moins 40% de protéines sont des protéines modifiées de lactosérum doux ne comprenant pas de cGMP ou un taux réduit de cGMP où le contenu en protéines de la formule n'est pas plus de 2g/100 kcal pour 1a préparation d'une formule pour développer une flore intestinale saine chez un nourrisson ou un enfant.
